# EUROPÄISCHE PATENTSCHRIFT

(11) **EP 0 453 885 B1**
(45) Veröffentlichungstag und Bekanntmachung des Hinweises auf die Patenterteilung: **16.03.1994**
(21) Anmeldenummer: 91105749.5
(22) Anmeldetag: 11.04.1991
(51) Int. Cl.: C07C 209/02, C07C 211/52, C07C 211/56, C07C 211/59

(54) **Verfahren zur Herstellung aromatischer Amine**
Process for the preparation of aromatic amines
Procédé pour la préparation d'amines aromatiques

(30) Priorität: 23.04.1990 PL 284896
(43) Veröffentlichungstag der Anmeldung: 30.10.1991
(73) Patentinhaber: BAYER AG, 51368 Leverkusen (DE)
(72) Erfinder: Makosza, Mieczyslaw, Prof. Dr., PL-01-312 Warszawa (PL); Bialecki, Maciej, PL-40-104 Katowice (PL)

(56) Entgegenhaltungen:
- THE JOURNAL OF ORGANIC CHEMISTRY, Band 53, Nr. 17, 19. August 1988, Seiten 3978-3982; A.R. KATRITZKY et al.: "Alkylaminonitrobenzenes by Vicarious Nucleophilic Amination with 4-(Alkylamino)-1,2,4-triazoles"
- ORGANIC SYNTHESES COLLECTIVE VOLUME, Nr. 3, 1955, Seite 664, Wiley, New York, US; C.C. PRICE et al.: "4-Nitro-1-Naphthylamine"
- THE JOURNAL OF ORGANIC CHEMISTRY, Band 51, Nr. 25, 12. Dezember 1986, Seiten 5038-5040; A. PADWA et al: "Direct Amination of Nitrobenzenes by Vicarious Nucleophilic Substitution"

## Beschreibung

Die Erfindung betrifft ein Verfahren zur Herstellung aromatischer Amine durch Aminierung elektrophiler Aromaten mit Sulfenamiden.

Aromatische Amine sind wichtige Zwischenprodukte für die Herstellung von Farbstoffen, Pflanzenschutzmitteln, Pharmazeutika und für die Fotoindustrie.

Die am häufigsten angewandte Methode zur Herstellung aromatischer Amine ist die Reduktion der Nitrogruppen der leicht zugänglichen Nitroaromaten. Auch die Umsetzung von Halogenaromaten mit Ammoniak oder Aminen führt zu aromatischen Aminen. Obwohl diese Verfahren in großem Umfang technisch angewandt werden, ergeben sich wegen der mehrstufigen Reaktion oft nicht zufriedenstellende Ausbeuten.

Auch die direkte Aminierung einiger heterocyclischer Aromaten, insbesondere Chinolin und Pyridin, mit Hilfe von Alkalimetallamid, vorzugsweise Natriumamid, ist bekannt (Tschitschibabin-Synthese). Leider ist die Anwendung auf wenige Heterocyclen beschränkt. Auch Aminierungen von Nitroaromaten mit Hydroxylamin (C.C. Price und S.-T. Voong, Org. Synth. Coll. Vol. III (1955) 664) und mit 4-Amino-1.2.4-triazol (A.R. Katrizky und K.S. Laurenzo, J. Org. Chem. 51 (1986) 5039 und 53 (1988) 3978) waren bekannt. Diese Reaktionen sind auf Sonderfälle beschränkt und/oder liefern Ausbeuten, die nicht zufriedenstellen können.

Überraschenderweise wurde nun ein allgemein anwendbares Verfahren zur direkten Aminierung elektrophiler Aromaten gefunden: Die Umsetzung mit Sulfenamiden führt glatt und in guten Ausbeuten zu den aromatischen Aminen.

Gegenstand der Erfindung ist also ein Verfahren zur Herstellung aromatischer Amine der Formel 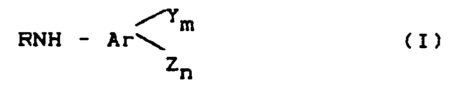
worin
- Ar: einen mono- oder polycyclischen, vorzugsweise mono- oder bicyclischen, aromatischen Rest mit 4 bis 16 C-Atomen, der auch 1 bis 2 Heteroatome aus der Reihe bestehend aus Stickstoff, Sauerstoff und Schwefel enthalten kann,
- R: Wasserstoff, C₁-C₄-Alkyl (gegebenenfalls durch Halogen oder C₁-C₄-Alkoxy substituiert), C₁-C₄-Alkenyl, C₅-C₁₂-Cycloalkyl, C₅-C₁₂-Aryl (gegebenenfalls durch Halogen, C₁-C₄-Alkyl oder C₁-C₄-Alkoxy substituiert), C₂-C₆-Acyl,
- Y: Nitro in 2- oder 4-Position, bezogen auf die Aminogruppe -NHR,
- Z: Halogen wie Fluor, Chlor, Brom, Iod; Cyano; C₁-C₄-Alkyl wie Methyl, Ethyl, Isopropyl; halogeniertes C₁-C₄-Alkyl wie Trifluormethyl, Dichlorfluormethyl; C₁-C₄-Alkoxy wie Methoxy und Ethoxy; C₁-C₄-Alkylmercapto (gegebenenfalls halogeniert) wie Methylmercapto und Trifluormethylmercapto; Di-C₁-C₄-alkylamino wie Dimethylamino und Diethylamino; C₁-C₄-Alkylsulfonyl wie Methylsulfonyl; Carboxyl oder Hydroxy und
- m: 1 oder 2 und
- n: Null, 1 oder 2 bedeuten.

Die Bedeutungen der Substituenten Z ist so zu verstehen, daß im Falle n = 2 die beiden Substituenten Z nicht gleich sein müssen.

Das erfindungsgemäße Verfahren wird so ausgeführt, daß man Verbindungen der Formel 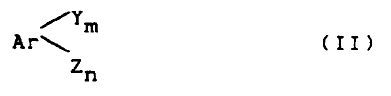
worin
- Y: Nitro bedeutet und die übrigen Symbole die oben angegebene Bedeutung haben,

mit organischen Sulfenamiden in Gegenwart von Basen umsetzt.

Ar bedeutet in Formel (I) einen (m+n+1)-bindigen, in Formel (II) einen (m+n)-bindigen Rest, vorzugsweise einen Benzol-, Naphthalin-, Pyridin-, Chinolin- oder Thiophenrest.

Bevorzugte Ausgangsverbindungen (II) umfassen also o-Chlornitrobenzol, m-Nitrobenzonitril, m-Trifluormethylnitrobenzol, 1- und 2-Nitronaphthaline, 2- und 3-Nitrothiophene, 2-Nitrofurane, N-alkylierte und N-arylierte 2- und 3-Nitropyrrole, 2, 3-und 4-Nitropyridine, 4-Ethoxy-3-nitropyridin, 5-, 6- und 8-Nitrochinoline.

Als organische Sulfenamide können praktisch alle zugänglichen stabilen Sulfenamide eingesetzt werden, also alle zugänglichen stabilen Verbindungen, die pro Molekül mindestens einmal die Gruppe -NH-S- enthalten. Bevorzugte Sulfenamide umfassen beispielsweise Benzthiazyl-2-sulfenamid, N-Phenyl-benzolsulfenamid, Benzthiazyl-2-cyclohexylsulfenamid, Benzthiazyl-2-tert.-butylsulfenamid, N,N-Dimethyl- und N,N-Diethyl-thiocarbamoylsulfenamid und N,N-Tetramethylen-thiocarbamoylsulfenamid.

Das Molverhältnis Ausgangsverbindung II/Sulfenamid beträgt im allgemeinen 0,8 bis 1,2, vorzugsweise 0,9 bis 1,1 und insbesondere etwa 1:1.

Es wird angenommen, daß die Reaktion nach folgendem Mechanismus abläuft:
Das durch Abstraktion eines Stickstoff-ständigen Wasserstoffatoms entstandene Sulfenamid-Anion bildet mit dem Aromaten II ein Addukt, woraus dann baseninduziert HSX eliminiert wird. Daraus ergibt sich, daß die Reaktion durch Anwesenheit basischer Mittel erst möglich wird.

Bevorzugte basische Mittel umfassen beispielsweise Alkalihydroxide, -hydride, -amide und -C₁-C₄-alkoholate wie z.B. Natrium- und Kaliumhydroxid, Natriumhydrid, Natriumamid, Lithiumdiisopropylamid, Natriummethylat, Natriumethylat und Kalium-tert.-butylat.

Das basische Mittel wird man in der Regel in einer Menge von mindestens 2 Mol, vorzugsweise 2 bis 7 Mol, insbesondere 2 bis 3,5 Mol, pro Mol Ausgangsverbindung II oder pro Mol Sulfenamid einsetzen (falls Ausgangsverbindung II und Sulfenamid nicht in äquimolarem Verhältnis eingesetzt werden: bezogen auf die unterschüssige Komponente).

Das erfindungsgemäße Verfahren kann in flüssigem Ammoniak oder in aprotischen organischen Lösungsmitteln durchgeführt werden. Bevorzugte aprotische organische Lösungsmittel umfassen beispielsweise Dimethylformamid, Dimethylsulfoxid, Tetrahydrofuran, Dimethoxyethan, Toluol und deren Mischungen.

Bei der erfindungsgemäßen Aminierung tritt die Aminogruppe -NHR meistens in die p-Position (bezogen auf den Substituenten Y) ein. Ist die p-Position bereits besetzt, so wird die Aminogruppe in der Regel in die o-Position gelenkt. Gelegentlich ist es auch möglich, durch die Wahl der Reaktionsbedingungen, insbesondere des Lösungsmittels und des basischen Mittels, die Aminogruppe -NHR in die gewünschte Position zu dirigieren: Beispielsweise führt die Reaktion von N,N-Tetramethylenthiocarbamoylsulfenamid mit 1-Nitronaphthalin in Gegenwart von Kaliumhydroxid in Dimethylsulfoxid zur Substitution in 4-Position, während die Reaktion von Benzthiazyl-2-sulfenamid mit 1-Nitronaphthalin in Gegenwart von Kalium-tert.-butylat in Dimethylformamid zur Substitution in der 2-Position führt.

Das erfindungsgemäße Verfahren kann bei Temperaturen von -50 bis +100°C, vorzugsweise von -35 bis +30°C durchgeführt werden.

Die dem erfindungsgemäßen Verfahren zugrundeliegende Umsetzung ist - u. a. abhängig von Reaktionstemperatur, Reaktivität der eingesetzten Verbindungen und Größe des Ansatzes - in wenigen Minuten bis einigen Stunden beendet. Das Fortschreiten der Reaktion kann beispielsweise dünnschichtchromatographisch verfolgt werden.

Nach Beendigung der Umsetzung kann der Ansatz in Wasser gegossen werden, das gelöstes Salz. z.B. Kochsalz oder Ammoniumchlorid, enthalten kann, um die Löslichkeit der Amine I in der wäßrigen Phase herabzusetzen. Aus der organischen Phase können dann die aromatischen Amine auf an sich bekannte Weise gewonnen werden. Zwecks weiterer Reinigung kann sich eine Umkristallisation und/oder eine chromatographische Reinigung anschließen.

Bei den Prozentangaben der nachfolgenden Beispiele handelt es sich um Gewichtsprozente.

### Beispiele

### Beispiel 1

### Herstellung von 4-Nitro-1-naphthylamin:

3,5 g 1-Nitronaphthalin und 3,3 g N,N-Tetramethylenthiocarbamoylsulfenamid, gelöst in 15 ml Dimethylsulfoxid, werden tropfenweise zu einer heftig gerührten Suspension von 6 g Kaliumhydroxid in 50 ml Dimethylsulfoxid gegeben, wobei die Temperatur bei 20 bis 25°C gehalten wird. Nach Vollendung der Zugabe wird noch 60 Minuten lang gerührt, und die Mischung wird in 400 ml gesättigte wäßrige Ammoniumchlorid-Lösung gegossen, und das Gemisch wird mit Methylenchlorid extrahiert. Nach dem Trocknen und der Entfernung des Lösungsmittels wird 4-Nitro-1-naphthylamin mit einem Schmelzpunkt von 190 bis 192°C erhalten. Die Ausbeute beträgt 2,9 g (77 %). Zusätzlich wird 1-Nitro-2-naphthylamin in einer Ausbeute von ca. 2 % erhalten.

### Beispiel 2

### Herstellung von 1-Nitro-2-naphthylamin:

3,5 g 1-Nitronaphthalin und 3,7 g Benzthiazyl-2-sulfenamid, gelöst in 30 ml Dimethylformamid, werden tropfenweise zu einer gerührten Lösung von 6 g Kalium-tert.-butylat in 60 ml Dimethylformamid gegeben, während die Temperatur auf 20 bis 25°C gehalten wird. Nach weiterem 15-minütigen Rühren wird die Mischung in 400 ml gesättigte wäßrige Ammoniumchlorid-Lösung gegossen und weiterhin wie in Beispiel 1 behandelt. 1-Nitro-2-naphthylamin mit einem Schmelzpunkt von 123 bis 125°C wird in einer Ausbeute von 2,9 g (72 %) erhalten. Zusätzlich werden ca. 8 % 4-Nitro-1-naphthylamin erhalten.

### Beispiel 3

### Herstellung von 3-Chlor-4-nitroanilin:

Aus 0,32 g o-Chlornitrobenzol, 0,33 g N,N-Tetramethylenthiocarbamoylsulfenamid und 0,6 g Kalium-tert.-butylat wird analog dem Verfahren aus Beispiel 2 3-Chlor-4-nitroanilin mit einem Schmelzpunkt von 156 bis 159°C in einer Ausbeute von 0,25 g (72 %) erhalten.

### Beispiel 4

### Herstellung von 2-Amino-5-nitrobenzonitril:

Aus 0,3 g m-Nitrobenzonitril, 0,33 g N,N-Tetramethylenthiocarbamoylsulfenamid und 0,6 g pulverisiertem Kaliumhydroxid wird analog dem Verfahren aus Beispiel 1 2-Amino-5-nitrobenzonitril mit einem Schmelzpunkt von 202 bis 204°C erhalten. Die Ausbeute beträgt 0,26 g (78 %).

### Beispiel 5

### Herstellung von N-Phenyl-4-nitro-1-naphthylamin:

Aus 0,35 g 1-Nitronaphthalin, 0,4 g N-Phenylbenzolsulfenamid und 0,6 g pulverförmigem Kaliumhydroxid wird analog dem Verfahren aus Beispiel 1 N-Phenyl-4-nitro-1-naphthylamin mit einem Schmelzpunkt von 158°C erhalten; die Ausbeute beträgt 0,32 g (61 %).

### Beispiel 6

### Herstellung von 2-Amino-4-ethoxy-5-nitropyridin:

Aus 0,34 g 4-Ethoxy-3-nitropyridin, 0,33 g N,N-Tetramethylenthiocarbamoylsulfenamid und 0,6 g Kalium-tert.-butylat wird analog dem Verfahren aus Beispiel 2 2-Amino-4-ethoxy-5-nitropyridin mit einem Schmelzpunkt von 215 bis 217°C erhalten; die Ausbeute beträgt 0,28 g (75 %).

### Beispiel 7

### Herstellung von N-Phenyl-5-nitro-2- und -3-thienylamin:

2,9 g 2-Nitrothiophen und 4 g N-Phenylbenzolsulfenamid, gelöst in 10 ml Dimethylformamid, werden tropfenweise zu einer gerührten Lösung aus 6 g Kalium-tert.-butylat in 60 ml Dimethylformamid gegeben, während die Temperatur bei ca. -20°C gehalten wird. Die Mischung wird für weitere 15 Minuten gerührt; dann wird die Mischung mit einem Überschuß an verdünnter Salzsäure versetzt und anschließend so behandelt, wie es in Beispiel 1 beschrieben ist. Das rohe Produkt wird durch Säulenchromatographie getrennt; man erhält N-Phenyl-5-nitro-2-thienylamin (Schmelzpunkt 186°C, Ausbeute 1,8 g, 42 %) und N-Phenyl-2-nitro-3-thienylamin (Schmelzpunkt 105°C, Ausbeute 0,7 g, 15 %).

### Beispiel 8

### Herstellung von 4-Nitro-2-trifluormethyl-anilin

Eine Lösung aus 3.82 g (0,02 Mol) 3-Trifluormethylnitrobenzol, 3,3 g (0,02 Mol) N,N-Diethylthiocarbamoylsulfenamid und 0,8 ml (0,02 Mol) Methanol wird tropfenweise in eine gerührte Suspension von 4 g (0,1 Mol) Natriumhydroxid in 40 ml trockenem flüssigem Ammoniak gegeben. Durch Kühlung von außen wird die Innentemperatur während der Zugabe so gehalten, daß der Ammoniak leicht unter Rückfluß gehalten wird. Nach Beendigung der Zugabe (10 Minuten) wird die Mischung 6 Stunden bei -30 bis -33°C gerührt und der Ammoniak verdampft, bis die Mischungstemperatur 0°C erreicht. Die Mischung wird dann auf -10°C abgekühlt, und dann werden 120 ml Wasser zugetropft, wobei die Temperatur unter +15°C gehalten wird. Zum Schutz gegen Luft werden der Mischung 3 ml Petrolether zugesetzt. Nach 15 Minuten Rühren bleibt der Ansatz über Nacht stehen.

Der Niederschlag wird abfiltriert und an der Luft getrocknet. Man erhält 3,5 g (85% d. Th.) Produkt; Fp. 85-89°C. Es besteht zu 92-93% aus dem gewünschten 4-Nitro-2-trifluormethyl-anilin und ca. 7% 4-Nitro-3-trifluormethylanilin, das sich aus 2-Trifluormethylnitrobenzol (= Verunreinigung des Ausgangsprodukts) gebildet hat. Laut ¹H-NMR (200Hz) enthält das Produkt weniger als 1% Disulfid.

Das wäßrige Filtrat wird innerhalb von 20 min bei -12 bis -15°C zu einer gerührten Mischung aus 20 ml 25%-igem wäßrigem Ammoniak und 23 ml 0,95n NaOCl getropft. Nach weiterem Rühren (30 min) bei dieser Temperatur wird der Feststoff abfiltriert, in Methylenchlorid gelöst, die Lösung mit wasserfreiem Natriumsulfat getrocknet und das Lösungsmittel abgezogen. Man erhält 2,8 g N,N-Diethylthiocarbamoylsulfenamid (85% d. Th.) als sich verfestigendes Öl.

Ähnliche Ergebnisse erhält man unter Verwendung von N,N-Dimethylthiocarbamoylsulfenamid.

## Patentansprüche

1. Verfahren zur Herstellung aromatischer Amine der Formel 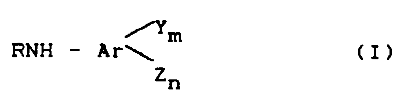 worin
Ar einen mono- oder polycyclischen aromatischen Rest mit 4 bis 16 C-Atomen, der auch 1 bis 2 Heteroatome aus der Reihe bestehend aus Stickstoff, Sauerstoff und Schwefel enthalten kann,
R Wasserstoff, C₁-C₄-Alkyl (gegebenenfalls durch Halogen oder C₁-C₄-Alkoxy substituiert), C₁-C₄-Alkenyl, C₅-C₁₂-Cycloalkyl, C₅-C₁₂-Aryl (gegebenenfalls durch Halogen, C₁-C₄-Alkyl oder C₁-C₄-Alkoxy substituiert),
Y Nitro in 2- oder 4-Position, bezogen auf die Aminogruppe -NHR,
Z Halogen, Cyano, gegebenenfalls halogeniertes C₁-C₄-Alkyl, C₁-C₄-Alkoxy, gegebenenfalls halogeniertes C₁-C₄-Alkylmercapto, Di-C₁-C₄-alkylamino, C₁-C₄-Alkylsulfonyl, Carboxyl oder Hydroxy und
m 1 oder 2 und
n Null, 1 oder 2 bedeuten,
wonach man Verbindungen der Formel 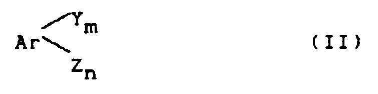 worin
Y Nitro bedeutet und die übrigen Symbole die oben angegebene Bedeutung haben,
mit organischen Sulfenamiden in Gegenwart von Basen umsetzt.

2. Verfahren nach Anspruch 1, wonach man die Umsetzung in aprotischen organischen Lösungsmitteln oder in flüssigem Ammoniak ausführt.

3. Verfahren nach Anspruch 1, wonach man die Umsetzung bei Temperaturen von -50 bis +100°C ausführt.

4. Verfahren nach Anspruch 1, wonach man die Basen in einer Menge von 2 bis 7 Mol pro Mol Ausgangsverbindung (II) einsetzt.

5. Verfahren nach Anspruch 1, wonach man die Ausgangsverbindungen (II) und die Sulfenamide in einem Molverhältnis von 0,8 bis 1,2 umsetzt.

## Claims

1. Process for preparing aromatic amines of the formula 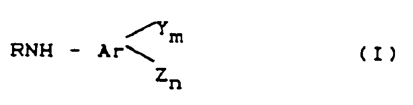 in which
Ar is a mono- or polycyclic aromatic radical having 4 to 16 C atoms, which radical may also contain 1 or 2 hetero atoms from the series consisting of nitrogen, oxygen and sulphur,
R is hydrogen, C₁-C₄-alkyl (optionally substituted by halogen or C₁-C₄-alkoxy), C₁-C₄-alkenyl, C₅-C₁₂-cycloalkyl, C₅-C₁₂-aryl (optionally substituted by halogen, C₁-C₄-alkyl, or C₁-C₄-alkoxy),
Y is nitro in the 2 or 4 position, relative to the amino group -NHR,
Z is halogen, cyano, optionally halogenated C₁-C₄-alkyl, C₁-C₄-alkoxy, optionally halogenated C₁-C₄-alkylmercapto, di-C₁-C₄-alkylamino, C₁-C₄-alkylsulphonyl, carboxyl or hydroxyl, and
m is 1 or 2, and
n is zero, 1 or 2,
according to which compounds of the formula 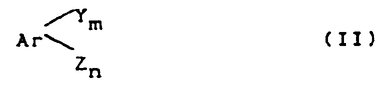 in which
Y is nitro and the remaining symbols have the abovementioned meaning,
are reacted with organic sulphenamides in the presence of bases.

2. Process according to Claim 1, wherein the reaction is carried out in aprotic organic solvents or in liquid ammonia.

3. Process according to Claim 1, wherein the reaction is carried out at temperatures of -50 to +100°C.

4. Process according to Claim 1, wherein the bases are used in an amount of 2 to 7 mol per mole of starting compound (II).

5. Process according to Claim 1, wherein the starting compounds (II) and the sulphenamides are reacted in a molar ratio of 0.8 to 1.2.

## Revendications

1. Procédé de préparation d'amines aromatiques de formule : 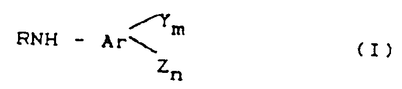 dans laquelle
Ar représente un radical aromatique mono- ou polycyclique en C₄-C₁₆ qui peut également contenir 1 à 2 hétéroatomes choisis parmi l'azote, l'oxygène et le soufre,
R représente l'hydrogène, un groupe alkyle en C₁-C₄ (éventuellement substitué par des halogènes ou des groupes alcoxy en C₁-C₄), un groupe alcényle en C₁-C₄, cycloalkyle en C₅-C₁₂, aryle en C₅-C₁₂ (éventuellement substitué par des halogènes, des groupes alkyles en C₁-C₄ ou alcoxy en C₁-C₄),
Y représente un groupe nitro en position 2 ou 4 par rapport au groupe amino -NHR,
Z représente un halogène, un groupe cyano, un groupe alkyle en C₁-C₄ éventuellement halogéné, un groupe alcoxy en C₁-C₄, un groupe alkylmercapto en C₁-C₄ éventuellement halogéné, un groupe di-(alkyle en C₁-C₄)-amino, alkylsulfonyle en C₁-C₄, carboxyle ou hydroxy et
m est égal à 1 ou 2 et
n est égal à 0, 1 ou 2,
dans lequel on fait réagir des composés de formule: 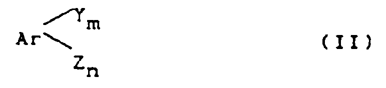 dans laquelle
Y représente un groupe nitro et les autres symboles ont les significations indiquées ci-dessus,
avec des sulfénamides organiques en présence de bases.

2. Procédé selon la revendication 1, dans lequel on effectue la réaction dans des solvants organiques aprotoniques ou dans l'ammoniac liquéfié.

3. Procédé selon la revendication 1, dans lequel on effectue la réaction à des températures de -50 à +100°C.

4. Procédé selon la revendication 1, dans lequel on met en oeuvre les bases en quantité de 2 à 7 mol par mole du composé de départ II.

5. Procédé selon la revendication 1, dans lequel on fait réagir les composés de départ II et les sulfénamides à un rapport molaire de 0,8 à 1,2.
